# EUROPEAN PATENT SPECIFICATION

(11) **EP 3 242 705 B1**
(45) Date of publication and mention of the grant of the patent: **19.06.2019**
(21) Application number: 16700939.8
(22) Date of filing: 06.01.2016
(51) Int. Cl.: A61M 25/01, A61M 25/06, A61M 25/00, A61B 17/12

(54) **MEDICAL DEVICE WITH A REMOVABLE LINER**
MEDIZINISCHE VORRICHTUNG MIT ENTFERNBARER AUSKLEIDUNG
DISPOSITIF MÉDICAL COMPORTANT UN REVÊTEMENT AMOVIBLE

(30) Priority: 08.01.2015 US 201562101195 P
(43) Date of publication of application: 15.11.2017
(73) Proprietor: Boston Scientific Scimed, Inc., Maple Grove, MN 55311 (US)
(72) Inventor: SMITH, Mark S., Coon Rapids, Minnesota 55448 (US); SPENCER, Steven M., Minneapolis, Minnesota 55406 (US); OLSON, Ross A., Anoka, Minnesota 55303 (US); HARRISON, Kent D., Maple Grove, Minnesota 55311 (US)
(74) Representative: Vossius & Partner Patentanwälte Rechtsanwälte mbB
(86) International application number: PCT/US2016/012368
(87) International publication number: WO 2016/112123

(56) References cited:
- US-A1- 2003 216 685
- US-A1- 2006 064 056
- US-A1- 2012 226 341
- US-A1- 2014 018 773

## Description

### Cross-Reference to Related Applications

This application claims priority under 35 U.S.C. §119 to U.S. Provisional Application Serial No. 62/101,195, filed January 8, 2015.

### Technical Field

The present disclosure pertains to medical devices, and methods for manufacturing medical devices. More particularly, the present disclosure pertains to medical devices with a removable liner.

### Background

A wide variety of intracorporeal medical devices have been developed for medical use, for example, intravascular use. Some of these devices include guidewires, catheters, and the like. These devices are manufactured by any one of a variety of different manufacturing methods and may be used according to any one of a variety of methods. Of the known medical devices and methods, each has certain advantages and disadvantages. There is an ongoing need to provide alternative medical devices as well as alternative methods for manufacturing and using medical devices. A prior art device is shown in US 2006/064056, which discloses a medical device system comprising a catheter shaft having a proximal end, a removable liner extending through the catheter shaft, the removable liner being designed to deliver a medical substance to a target region, wherein the removable liner is designed to be removed from the catheter shaft if the removable liner becomes plugged and a replacement liner, wherein the replacement liner is designed to be inserted within the catheter shaft after the removal of the removable liner.

### Brief Summary

This disclosure provides design, material, manufacturing method, and use alternatives for medical devices and/or medical device systems. The invention is defined by the appended claims. Subject matter referred as embodiments and/or inventions which are not claimed are not part of the invention. An example medical device system is disclosed. The system comprises:
a catheter shaft having a proximal end;
a removable liner extending through the catheter shaft;
wherein the removable liner is designed to deliver a medical substance to a target region;
wherein the removable liner is designed to be removed from the catheter shaft if the removable liner becomes plugged;
a replacement liner;
wherein the replacement liner is designed to be inserted within the catheter shaft after the removal of the removable liner; and
a support member disposed along the replacement liner.

Alternatively or additionally to any of the embodiments above, the catheter shaft includes a first hub and wherein the removable liner includes a second hub designed to engage the first hub.

Alternatively or additionally to any of the embodiments above, the first hub, the second hub, or both include a threaded connector.

Alternatively or additionally to any of the embodiments above, the catheter shaft includes a reinforcing member.

Alternatively or additionally to any of the embodiments above, the removable liner includes a reinforcing member.

Alternatively or additionally to any of the embodiments above, the replacement liner defines a lumen and wherein the support member includes a delivery stylet disposed within the lumen.

Alternatively or additionally to any of the embodiments above, the support member includes a breakaway sheath disposed along an outer surface of the replacement liner.

Alternatively or additionally to any of the embodiments above, the removable liner, the replacement liner, or both are designed to shift between a collapsed configuration and a non-collapsed configuration.

Alternatively or additionally to any of the embodiments above, the removable liner, the replacement liner, or both include a temporary distal seal member.

An example medical device is disclosed. The medical device comprises:
a tubular member having a proximal end region and a distal end region;
a first hub member coupled to the proximal end region;
a removable liner extending through the tubular member;
wherein the removable liner is free from attachment with the tubular member;
a second hub member coupled to the removable liner;
wherein the first hub member and the second hub member are designed to be detachably nested with one another; and
wherein the removable liner is designed to be removed from the tubular member so that a second liner may be disposed within the tubular member.

Alternatively or additionally to any of the embodiments above, the tubular member includes a reinforcing member.

Alternatively or additionally to any of the embodiments above, the removable liner includes a reinforcing member.

Alternatively or additionally to any of the embodiments above, the removable liner includes a lubricious coating.

Alternatively or additionally to any of the embodiments above, the removable liner is designed to shift between a collapsed configuration and a non-collapsed configuration.

Alternatively or additionally to any of the embodiments above, the first hub member, the second hub member, or both include a threaded connector.

Alternatively or additionally to any of the embodiments above, the removable liner includes a temporary distal seal member.

Alternatively or additionally to any of the embodiments above, an embolic material delivery device is releasably attached to the removable liner.

An example for treating a body lumen is disclosed. The method comprises:
positioning an embolic material delivery device adjacent to a target region;
wherein the embolic material delivery device comprises:
   a catheter shaft,
   a removable liner extending through the catheter shaft, and
   wherein the removable liner is free from attachment with the catheter shaft;
advancing a first medical substance through the removable liner to a position adjacent to the target region;
removing the removable liner from the catheter shaft;
advancing a replacement liner through the catheter shaft; and
advancing a second medical substance through the replacement liner, wherein the second medical substance is the same as or different from the first medical substance.

Alternatively or additionally to any of the embodiments above, the replacement liner is prefilled with the second medical substance.

Alternatively or additionally to any of the embodiments above, the method may include advancing a third medical substance through the catheter shaft.

The above summary of some embodiments is not intended to describe each disclosed embodiment or every implementation of the present disclosure. The Figures, and Detailed Description, which follow, more particularly exemplify these embodiments.

### Brief Description of the Drawings

The disclosure may be more completely understood in consideration of the following detailed description in connection with the accompanying drawings, in which:
Figure 1 is a cross-sectional side view of an example medical device system disposed in a body lumen;
Figure 2 is a cross-sectional side view of an example medical device system with a removable liner removed from the system;
Figure 3 is a cross-sectional side view illustrating the delivery of a replacement liner;
Figure 3A is a side view of a portion of an example support member;
Figure 4 is a cross-sectional side view of an example medical device system with a replacement liner;
Figure 5 is an example transverse cross-sectional view of a portion of an example medical device system;
Figure 6 is an example transverse cross-sectional view of a portion of an example medical device system;
Figure 7 is an example transverse cross-sectional view of a portion of an example medical device system;
Figure 8 is an example transverse cross-sectional view of a portion of an example medical device system;
Figure 9 is a cross-sectional side view of an example medical device system;
Figure 10 is a cross-sectional side view of an example medical device system; and
Figure 11 is a cross-sectional side view of an example medical device system.

While the disclosure is amenable to various modifications and alternative forms, specifics thereof have been shown by way of example in the drawings and will be described in detail. It should be understood, however, that the intention is not to limit the invention to the particular embodiments described. On the contrary, the intention is to cover all modifications, equivalents, and alternatives falling within the spirit and scope of the disclosure.

### Detailed Description

For the following defined terms, these definitions shall be applied, unless a different definition is given in the claims or elsewhere in this specification.

All numeric values are herein assumed to be modified by the term "about", whether or not explicitly indicated. The term "about" generally refers to a range of numbers that one of skill in the art would consider equivalent to the recited value (e.g., having the same function or result). In many instances, the terms "about" may include numbers that are rounded to the nearest significant figure.

The recitation of numerical ranges by endpoints includes all numbers within that range (e.g. 1 to 5 includes 1, 1.5, 2, 2.75, 3, 3.80, 4, and 5).

As used in this specification and the appended claims, the singular forms "a", "an", and "the" include plural referents unless the content clearly dictates otherwise. As used in this specification and the appended claims, the term "or" is generally employed in its sense including "and/or" unless the content clearly dictates otherwise.

It is noted that references in the specification to "an embodiment", "some embodiments", "other embodiments", etc., indicate that the embodiment described may include one or more particular features, structures, and/or characteristics. However, such recitations do not necessarily mean that all embodiments include the particular features, structures, and/or characteristics. Additionally, when particular features, structures, and/or characteristics are described in connection with one embodiment, it should be understood that such features, structures, and/or characteristics may also be used in connection with other embodiments whether or not explicitly described unless clearly stated to the contrary.

The following detailed description should be read with reference to the drawings in which similar elements in different drawings are numbered the same. The drawings, which are not necessarily to scale, depict illustrative embodiments and are not intended to limit the scope of the invention.

Medical devices including catheters and/or microcatheters may be used to diagnose and/or treat a number of different conditions. For example, in certain scenarios it may be desirable to utilize a microcatheter to perform an embolization procedure. Such a procedure may include the infusion of an embolic material (e.g., an embolic glue, gel, foam, or the like) into a body lumen in order to seal off or otherwise block the body lumen (e.g., block a blood vessel, fill an aneurism, etc.). In some instances, the embolic material itself may be tacky, sticky, or clumpy. This may aid in the function of the embolic material. However, when such materials are used, the microcatheter used to deliver the materials could become blocked or otherwise obstructed. When this happens, the microcatheter may be removed from the body lumen and replaced with a new device. While replacing the medical device can be carried out by clinician, such a procedure may be time consuming and/or technically challenging. Disclosed herein are medical devices and/or medical device systems that allow a plugged or clogged microcatheter to be efficiently "unplugged" without having to replace the entire device. Some additional details of the medical devices/systems are disclosed herein.

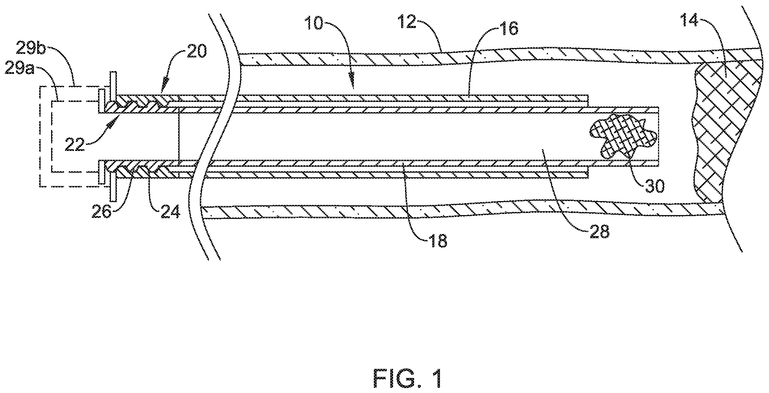

Figure 1 is an example medical device system 10 disposed within a blood vessel 12. System 10 may be used to deliver a medical substance 14 to a target region (e.g., along blood vessel 12). For example, system 10 may be used to deliver an embolic material, fill an aneurism, deliver a therapeutic agent or drug, a dye (e.g., an angiographic dye), or the like. System 10 may include a catheter shaft 16. Catheter shaft 16 may have a variety of shapes, lengths, sizes, and/or configurations. For example, catheter shaft 16 may be a microcatheter shaft. A microcatheter shaft may be understood as a catheter shaft have a relatively small outer diameter (e.g., about 3 Fr or less; about 1 mm or less (about 0.039 inches or less)). In other embodiments, catheter shaft 16 may have other sizes including an outer diameter similar to other intravascular catheters. A liner 18 may be disposed within catheter shaft 16. Rather than being another catheter or separate device merely extending through catheter shaft 16, liner 18 may take the form of a sleeve that extends along the inner surface of catheter shaft 16. In some instances, liner 18 has a distal end that terminates at the distal end of catheter shaft 16. In other instances, the distal end of liner 18 may extend distally beyond the distal end of catheter shaft 16 or may be positioned proximally of the distal end of catheter shaft 16. A first hub member 20 may be disposed at the proximal end of catheter shaft 16. A second hub member 22 may be disposed at the proximal end of liner 18. Hub member 20 may be capable of engaging with hub member 22. For example, hub member 20 may have an internal thread or connector 24 that is designed to threadably engage an external thread or connector 26 along hub member 22. The threaded connection may be similar to or otherwise take the form of a luer connector.

In general, medical substance 14 may be delivered through catheter shaft 16 by passing medical substance 14 through liner 18. For example, in some instances, a medical substance delivery member 29a such as a syringe may be coupled to liner 18 (e.g., at second hub member 22) as shown schematically in Figure 1. Alternatively or additionally, a medical substance delivery member 29b may be coupled to catheter shaft 16 (e.g., at first hub member 20). Medical substance 14 may also be delivered through catheter shaft 16. This may include delivering medical substance 14 between catheter shaft 16 and liner 18 or through the lumen of catheter shaft 16 (e.g., after the removal of liner 18).

As described herein, in some instances medical substance 14 may have a tacky or sticky consistency. Because of this, medical substance 14 could become stuck or clogged within, for example, liner 18. This may include medical substance 14 sticking to the outer surface of liner 18 (e.g., in instances where liner 18 extends distally beyond the distal end of catheter shaft 16), sticking to the inner surface of liner 18, clumping or clogging the lumen 28 of liner 18, or the like. In Figure 1, a portion 30 of medical substance 14 is shown lodged within and clogging liner 18.

Liner 18 of system 10 may be designed to be "removable". In other words, liner 18 may be removed from catheter shaft 16 so that it can be replaced with a second or "replacement" liner, for example when liner 18 is clogged with medical substance 14. In addition, in some situations it may be desirable to stop or limit infusions of medial substance 14 after some time or after a suitable quantity of medical substance 14 has been delivered. This may allow for a different medical substance 14 to be delivered, if desired. Therefore, in such situations it may be desirable to remove liner 18, even though liner 18 may not necessarily be clogged.

In order for liner 18 to be a replaceable liner 18, it may be desirable for liner 18 to be free from attachment with catheter shaft 16. For example, along the length of liner 18, liner 18 may be free from being bonded to, glued to, or otherwise secured with catheter shaft 16 (e.g., the inner surface of catheter shaft 16). In some instances, the only location where liner 18 and catheter shaft 16 are interconnected may be at hub members 20/22 (e.g., where hub member 20/22 are detachably connected). However, in other instances, other portions of liner 18 and catheter shaft 16 may be detachably connected including along the respective lengths thereof.

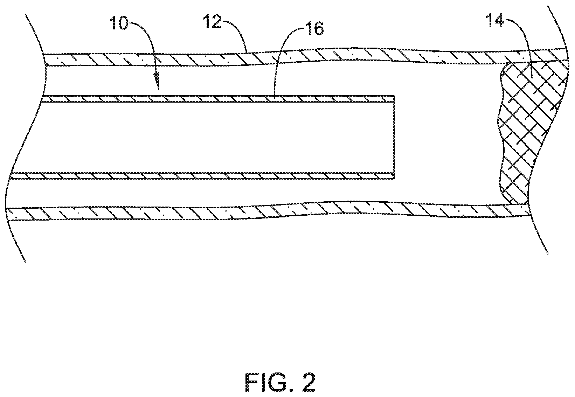

Figures 2-4 illustrate the process for replacement of liner 18 in system 10. For example, Figure 2 shows that when liner 18 becomes plugged or otherwise obstructed, liner may be removed. Removal of liner 18 may include grasping one or more of hub members 20/22, disengaging hub members 20/22 from one another (e.g., by unthreading, uncoupling, etc.), and proximally retracting liner 18 from the interior of catheter shaft 16.

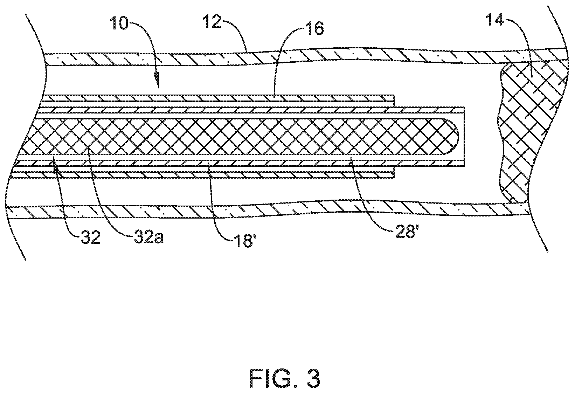

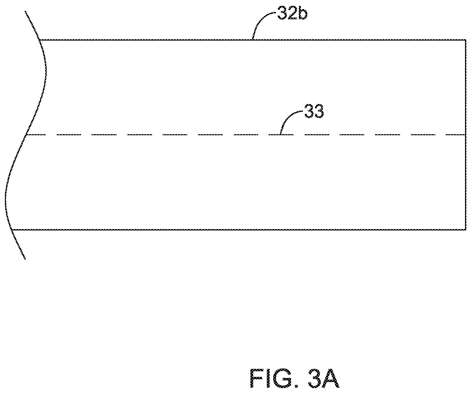

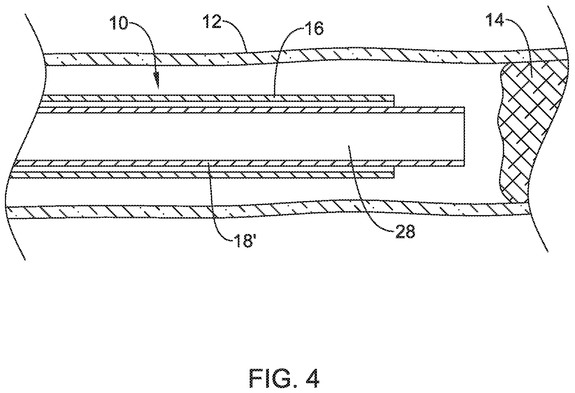

After the replaceable liner 18 has been removed from catheter shaft 16, a replacement liner 18' may be advanced through catheter shaft 16 as shown in Figure 3. Replacement liner 18' may be preloaded with a substance (e.g., medical substance 14, saline, or the like). In some instances, a support member 32a may be disposed along replacement liner 18' in order to aid in delivery. When replacement liner 18' is suitably positioned within catheter shaft 16, support member 32a may be removed as shown in Figure 4.

In at least some instances, support member 32 may take the form of a delivery stylet 32a disposed within the lumen 28' of replacement liner 18'. Stylet 32a may take the form of a shaft or wire that can be disposed within replacement liner 18'. In some instances, stylet 32a may simply sit within replacement liner 18' during advancement of replacement liner 18' through catheter shaft 16. In other instances, stylet 32a may engage a ridge, shoulder, radially inward projection, narrowed region, or the like within replacement liner 18' so that stylet 32a can "push" replacement liner 18' distally within catheter shaft 16. Other arrangements are contemplated. Stylet 32a may be a solid (e.g., non-tubular) structure or a tubular structure. In some instances, stylet 32a may be or resemble a guidewire.

In other instances, support member 32 may take the form of a breakaway sheath 32b as shown in Figure 3A. Breakaway sheath 32b can be disposed along an outer surface of replacement liner 18'. Before, during, or after disposing replacement liner 18' within catheter shaft 16, breakaway sheath 32b can be removed. Removal may include simply pulling breakaway sheath 32b off from replacement liner 18'. Alternatively, breakaway sheath 32b may include a plurality of cuts or slits, perforations, scores, or a line of weakness 33 that allows the breakaway sheath to be split, torn, cut, or otherwise removed from replacement liner 18'.

In at least some instances, replacement liner 18' may be prefilled with medical substance 14 and/or a different medical substance. For example, liner 18 may be used to deliver medical substance 14. Liner 18 may be removed and prefilled replacement liner 18' may be disposed within catheter shaft 16 so that another medical substance may be delivered. Prefilled replacement liner 18' may have sufficient strength so that prefilled replacement liner 18' may be disposed within catheter shaft 16 without the need of support member 32. However, in other instances, support member 32 (e.g., support member 32b) may be utilized with prefilled replacement liner 18'.

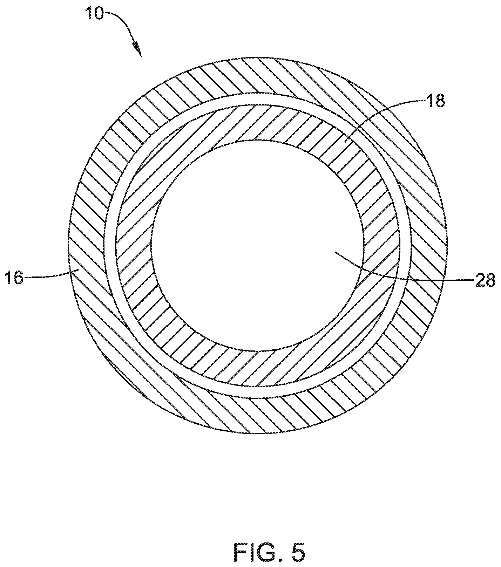

The form of the various components of system 10 may vary. For example, Figure 5 illustrates that catheter shaft 16, liner 18, or both may be formed from a single layer of material. However, this is not intended to be limiting. In some instances, the single layer of material may also include a coating (e.g., an exterior and/or interior coating). The coating may include a lubricious coating, a hydrophilic coating, or the like. In some of these and in other instances, catheter shaft 16, liner 18, or both may include a layer or coating that can leach a material that is designed to slow or prevent the curing or activating of medical substance 14. In other words, medical substance 14 may be passed through liner 18, and liner 18 may leach a material that slows or prevents the activation of medical substance 14 (e.g., while medical substance 14 is disposed within liner 18) until medical substance 14 exits the distal end of liner 18.

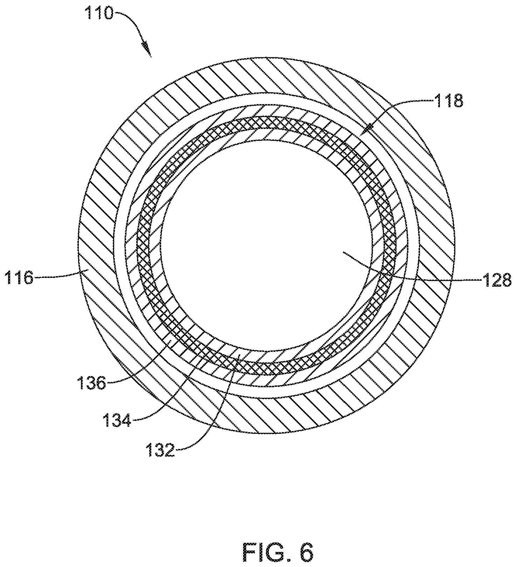

Other structural configurations and/or arrangements are contemplated for catheter shaft 16 and liner 18. For example, Figure 6 illustrates system 110 that may be similar in form and function to other systems disclosed herein. System 110 may include catheter shaft 116 and liner 118. Liner 118 defines lumen 128. In some instances, liner 118 may include a plurality of layers including an inner layer 132, a reinforcing member 134, and an outer layer 136. Reinforcing member 134 may include a coil, braid, mesh, or the like. Layers 132/136 may include a polymer or metal such as those disclosed herein. In some instances, each of layers 132/136 is formed from a single layer of material. In other instances, one or both of layers 132/136 include multiple layers of material and/or a coating (e.g., an interior coating, an exterior coating, a tie layer, or the like).

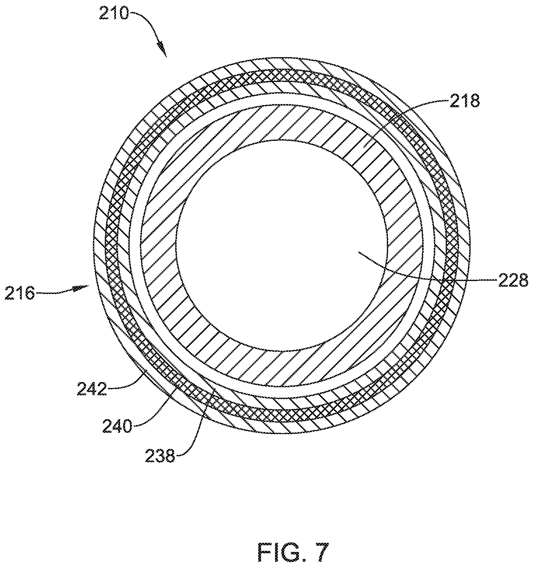

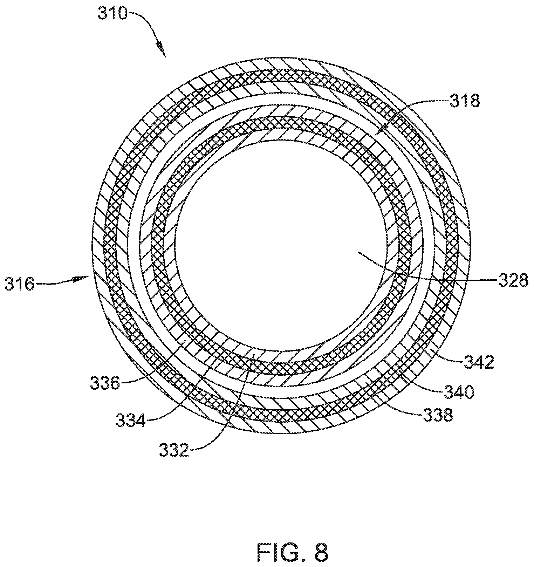

Figure 7-8 illustrate other structural configurations that are contemplated for the systems disclosed herein. For example, Figure 7 illustrates system 210 that may be similar in form and function to other systems disclosed herein. System 210 may include catheter shaft 216 and liner 218. Liner 218 defines lumen 228. In some instances, catheter shaft 216 may include a plurality of layers including an inner layer 238, a reinforcing member 240, and an outer layer 242. Figure 8 illustrates system 310 that may be similar in form and function to other systems disclosed herein. System 310 may include catheter shaft 316 and liner 318. Liner 318 defines lumen 328. In some instances, liner 318 may include a plurality of layers including inner layer 332, reinforcing member 334, and outer layer 336. Catheter shaft 316 may also include a plurality of layers including inner layer 338, reinforcing member 340, and outer layer 342. Collectively, these figures illustrate some of structural the configurations contemplated for the various systems disclosed herein.

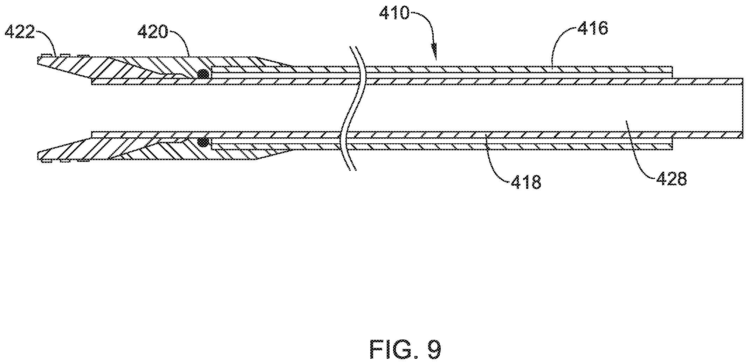

Figure 9 illustrates system 410 that may be similar in form and function to other systems disclosed herein. System 410 may include catheter shaft 416 and liner 418. Liner 418 defines lumen 428. In this example, catheter shaft 416 may include hub member 420. Liner 318 may include hub member 422. Hub member 420 and hub member 422 are designed to engage and detachably connect with one another. For example, hub members 420/422 are designed to mechanically interconnect with and/or otherwise nest with one another. This may include wedging hub member 422 within hub member 420.

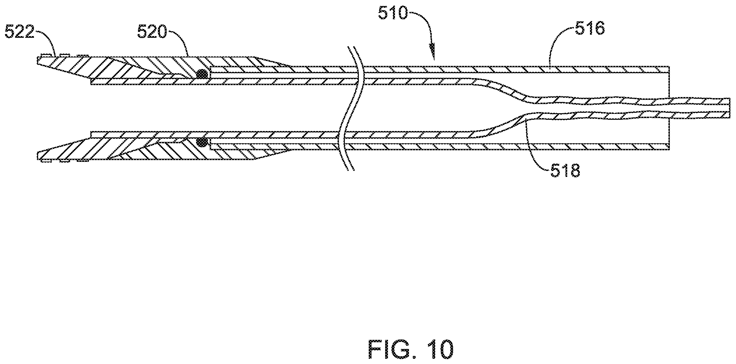

Figure 10 illustrates system 510 that may be similar in form and function to other systems disclosed herein. System 510 may include catheter shaft 516 and liner 518. In this instance, liner 518 may be a replacement liner that can be inserted into catheter shaft 516 after removal of the initial "removable liner". Liner 518 is designed to shift between a collapsed configuration (e.g., as shown in Figure 10) and a non-collapsed configuration (e.g., as shown in Figure 9). In some instances, the collapsed configuration of liner 518 may make it easier to insert liner 518 within catheter shaft 516. Once properly positioned, liner 518 can be expanded either actively (e.g., by infusing a material such as an embolic material through liner 518 to actively expand liner 518) or passively (e.g., where the material composition or folding configuration allows liner 518 to revert to a pre-defined non-collapsed configuration after positioning liner 518 within catheter shaft 516.

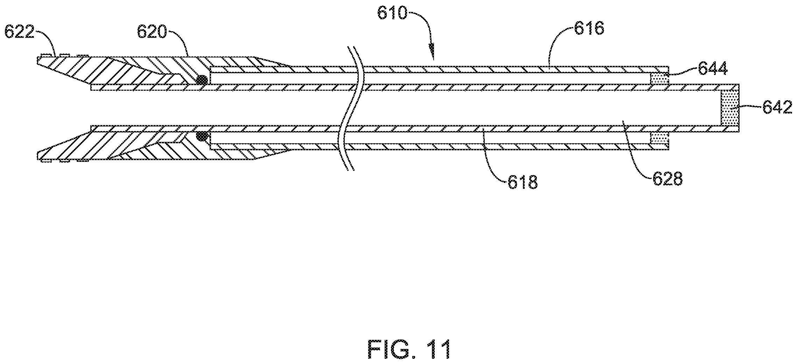

Figure 11 illustrates system 610 that may be similar in form and function to other systems disclosed herein. System 610 may include catheter shaft 616 and liner 618. Liner 618 defines lumen 628. In some instances, liner 618 is a replacement liner that may include a temporary seal member 642. Temporary seal 642 may allow liner 618 to be inserted into catheter shaft 616 (e.g., with a syringe or embolic fluid delivery device) while "sealed". In some instances, liner 618 may be filled with a fluid prior to insertion. The fluid may add column strength that aids in inserting and pushing liner 618 through catheter shaft 614. In some of these and in other embodiments, the fluid may be a fluid that "pre-treats" the target tissue. Once liner 618 is inserted, pressure can be applied with the syringe that is sufficient to break or disrupt seal 642, thereby allowing the embolic fluid to be passed through liner 618 to the target location. In some of these and in other embodiments, catheter shaft 616 may include a temporary seal member 644.

The materials that can be used for the various components of system 10 (and/or other systems disclosed herein) and the various tubular members disclosed herein may include those commonly associated with medical devices. For simplicity purposes, the following discussion makes reference to liner 18 and other components of system 10. However, this is not intended to limit the devices and methods described herein, as the discussion may be applied to other structures and components of the systems disclosed herein.

Liner 18 and/or other components of system 10 may be made from a metal, metal alloy, polymer (some examples of which are disclosed below), a metal-polymer composite, ceramics, combinations thereof, and the like, or other suitable material. Some examples of suitable polymers may include polytetrafluoroethylene (PTFE), ethylene tetrafluoroethylene (ETFE), fluorinated ethylene propylene (FEP), polyoxymethylene (POM, for example, DELRIN® available from DuPont), polyether block ester, polyurethane (for example, Polyurethane 85A), polypropylene (PP), polyvinylchloride (PVC), polyether-ester (for example, ARNITEL® available from DSM Engineering Plastics), ether or ester based copolymers (for example, butylene/poly(alkylene ether) phthalate and/or other polyester elastomers such as HYTREL® available from DuPont), polyamide (for example, DURETHAN® available from Bayer or CRISTAMID® available from Elf Atochem), elastomeric polyamides, block polyamide/ethers, polyether block amide (PEBA, for example available under the trade name PEBAX®), ethylene vinyl acetate copolymers (EVA), silicones, polyethylene (PE), Marlex high-density polyethylene, Marlex low-density polyethylene, linear low density polyethylene (for example REXELL®), polyester, polybutylene terephthalate (PBT), polyethylene terephthalate (PET), polytrimethylene terephthalate, polyethylene naphthalate (PEN), polyetheretherketone (PEEK), polyimide (PI), polyetherimide (PEI), polyphenylene sulfide (PPS), polyphenylene oxide (PPO), poly paraphenylene terephthalamide (for example, KEVLAR®), polysulfone, nylon, nylon-12 (such as GRILAMID® available from EMS American Grilon), perfluoro(propyl vinyl ether) (PFA), ethylene vinyl alcohol, polyolefin, polystyrene, epoxy, polyvinylidene chloride (PVdC), poly(styrene-*b*-isobutylene-*b-*styrene) (for example, SIBS and/or SIBS 50A), polycarbonates, ionomers, biocompatible polymers, other suitable materials, or mixtures, combinations, copolymers thereof, polymer/metal composites, and the like. In some embodiments the sheath can be blended with a liquid crystal polymer (LCP). For example, the mixture can contain up to about 6 percent LCP.

Some examples of suitable metals and metal alloys include stainless steel, such as 304V, 304L, and 316LV stainless steel; mild steel; nickel-titanium alloy such as linear-elastic and/or super-elastic nitinol; other nickel alloys such as nickel-chromium-molybdenum alloys (e.g., UNS: N06625 such as INCONEL® 625, UNS: N06022 such as HASTELLOY® C-22®, UNS: N10276 such as HASTELLOY® C276®, other HASTELLOY® alloys, and the like), nickel-copper alloys (e.g., UNS: N04400 such as MONEL® 400, NICKELVAC® 400, NICORROS® 400, and the like), nickel-cobalt-chromium-molybdenum alloys (e.g., UNS: R30035 such as MP35-N® and the like), nickel-molybdenum alloys (e.g., UNS: N10665 such as HASTELLOY® ALLOY B2®), other nickel-chromium alloys, other nickel-molybdenum alloys, other nickel-cobalt alloys, other nickel-iron alloys, other nickel-copper alloys, other nickel-tungsten or tungsten alloys, and the like; cobalt-chromium alloys; cobalt-chromium-molybdenum alloys (e.g., UNS: R30003 such as ELGILOY®, PHYNOX®, and the like); platinum enriched stainless steel; titanium; combinations thereof; and the like; or any other suitable material.

As alluded to herein, within the family of commercially available nickel-titanium or nitinol alloys, is a category designated "linear elastic" or "non-super-elastic" which, although may be similar in chemistry to conventional shape memory and super elastic varieties, may exhibit distinct and useful mechanical properties. Linear elastic and/or non-super-elastic nitinol may be distinguished from super elastic nitinol in that the linear elastic and/or non-super-elastic nitinol does not display a substantial "superelastic plateau" or "flag region" in its stress/strain curve like super elastic nitinol does. Instead, in the linear elastic and/or non-super-elastic nitinol, as recoverable strain increases, the stress continues to increase in a substantially linear, or a somewhat, but not necessarily entirely linear relationship until plastic deformation begins or at least in a relationship that is more linear that the super elastic plateau and/or flag region that may be seen with super elastic nitinol. Thus, for the purposes of this disclosure linear elastic and/or non-super-elastic nitinol may also be termed "substantially" linear elastic and/or non-super-elastic nitinol.

In some cases, linear elastic and/or non-super-elastic nitinol may also be distinguishable from super elastic nitinol in that linear elastic and/or non-super-elastic nitinol may accept up to about 2-5% strain while remaining substantially elastic (e.g., before plastically deforming) whereas super elastic nitinol may accept up to about 8% strain before plastically deforming. Both of these materials can be distinguished from other linear elastic materials such as stainless steel (that can also can be distinguished based on its composition), which may accept only about 0.2 to 0.44 percent strain before plastically deforming.

In some embodiments, the linear elastic and/or non-super-elastic nickel-titanium alloy is an alloy that does not show any martensite/austenite phase changes that are detectable by differential scanning calorimetry (DSC) and dynamic metal thermal analysis (DMTA) analysis over a large temperature range. For example, in some embodiments, there may be no martensite/austenite phase changes detectable by DSC and DMTA analysis in the range of about -60 degrees Celsius (°C) to about 120 °C in the linear elastic and/or non-super-elastic nickel-titanium alloy. The mechanical bending properties of such material may therefore be generally inert to the effect of temperature over this very broad range of temperature. In some embodiments, the mechanical bending properties of the linear elastic and/or non-super-elastic nickel-titanium alloy at ambient or room temperature are substantially the same as the mechanical properties at body temperature, for example, in that they do not display a super-elastic plateau and/or flag region. In other words, across a broad temperature range, the linear elastic and/or non-super-elastic nickel-titanium alloy maintains its linear elastic and/or non-super-elastic characteristics and/or properties.

In some embodiments, the linear elastic and/or non-super-elastic nickel-titanium alloy may be in the range of about 50 to about 60 weight percent nickel, with the remainder being essentially titanium. In some embodiments, the composition is in the range of about 54 to about 57 weight percent nickel. One example of a suitable nickel-titanium alloy is FHP-NT alloy commercially available from Furukawa Techno Material Co. of Kanagawa, Japan. Some examples of nickel titanium alloys are disclosed in U.S. Patent Nos. 5,238,004 and 6,508,803, which are incorporated herein by reference. Other suitable materials may include ULTANIUM™ (available from Neo-Metrics) and GUM METAL™ (available from Toyota). In some other embodiments, a superelastic alloy, for example a superelastic nitinol can be used to achieve desired properties.

In at least some embodiments, portions or all of liner 18 and/or other components of system 10 may also be doped with, made of, or otherwise include a radiopaque material. Radiopaque materials are understood to be materials capable of producing a relatively bright image on a fluoroscopy screen or another imaging technique during a medical procedure. This relatively bright image aids the user of system 10 in determining its location. Some examples of radiopaque materials can include, but are not limited to, gold, platinum, palladium, tantalum, tungsten alloy, polymer material loaded with a radiopaque filler, and the like. Additionally, other radiopaque marker bands and/or coils may also be incorporated into the design of system 10 to achieve the same result.

In some embodiments, a degree of Magnetic Resonance Imaging (MRI) compatibility is imparted into system 10. For example, liner 18 and/or other components of system 10 may be made of a material that does not substantially distort the image and create substantial artifacts (e.g., gaps in the image). Certain ferromagnetic materials, for example, may not be suitable because they may create artifacts in an MRI image. Liner 18 and/or other components of system 10 may also be made from a material that the MRI machine can image. Some materials that exhibit these characteristics include, for example, tungsten, cobalt-chromium-molybdenum alloys (e.g., UNS: R30003 such as ELGILOY®, PHYNOX®, and the like), nickel-cobalt-chromium-molybdenum alloys (e.g., UNS: R30035 such as MP35-N® and the like), nitinol, and the like, and others.

It should be understood that this disclosure is, in many respects, only illustrative. Changes may be made in details, particularly in matters of shape, size, and arrangement of steps without exceeding the scope of the disclosure. This may include, to the extent that it is appropriate, the use of any of the features of one example embodiment being used in other embodiments. The invention's scope is, of course, defined in the language in which the appended claims are expressed.

## Claims

1. A medical device system, comprising:
a catheter shaft (16) having a proximal end;
a removable liner (18) extending through the catheter shaft (16)
wherein the catheter shaft (16) includes a first hub (20) and wherein the removable liner includes a second hub (22) designed to engage the first hub;
wherein the removable liner is designed to deliver a medical substance to a target region;
wherein the removable liner is designed to be removed from the catheter shaft if the removable liner becomes plugged;
a replacement liner (18');
wherein the replacement liner is designed to be inserted within the catheter shaft after the removal of the removable liner; and
a support member (32a) disposed along the replacement liner.

2. The medical device system of claim 1, wherein the first hub, the second hub, or both include a threaded connector.

3. The medical device system of any one of claims 1-2, wherein the catheter shaft includes a reinforcing member.

4. The medical device system of any one of claims 1-3, wherein the removable liner includes a reinforcing member.

5. The medical device system of any one of claims 1-4, wherein the replacement liner defines a lumen and wherein the support member includes a delivery stylet disposed within the lumen.

6. The medical device system of any one of claims 1-4, wherein the support member includes a breakaway sheath disposed along an outer surface of the replacement liner.

7. The medical device system of any one of claims 1-6, wherein the removable liner, the replacement liner, or both are designed to shift between a collapsed configuration and a non-collapsed configuration.

8. The medical device system of any one of claims 1-7, wherein the removable liner, the replacement liner, or both include a temporary distal seal member.

9. The medical device system of any one of claims 1-8, wherein:
the first hub is coupled to a proximal end region of the catheter shaft;
the second hub is coupled to the removable liner; and
wherein the first hub and the second hub are designed to be detachably nested with one another.

10. The medical device system of any one of claims 1-9, wherein the removable liner is free from attachment with the catheter shaft.

11. The medical device system of any one of claims 1-10, wherein the removable liner includes a lubricious coating.

## Patentansprüche

1. Medizinisches Vorrichtungssystem, aufweisend:
einen Katheterschaft (16) mit einem proximalen Ende;
einen entfernbaren Liner (18), der sich durch den Katheterschaft (16) erstreckt;
wobei der Katheterschaft (16) ein erstes Verbindungsstück (20) aufweist und wobei der entfernbare Liner ein zweites Verbindungsstück (22) aufweist, das zum Eingreifen in das erste Verbindungsstück ausgelegt ist;
wobei der entfernbare Liner zum Abgeben einer medizinischen Substanz an eine Zielregion ausgelegt ist;
wobei der entfernbare Liner zum Entfernen aus dem Katheterschaft ausgelegt ist, wenn der entfernbare Liner verstopft wird;
einen Ersatzliner (18');
wobei der Ersatzliner zum Einführen in den Katheterschaft nach dem Entfernern des entfernbaren Liners ausgelegt ist, und
ein entlang des Ersatzliners angeordnetes Stützelement (32a).

2. Medizinisches Vorrichtungssystem nach Anspruch 1, wobei das erste Verbindungsstück, das zweite Verbindungsstück oder beide einen Gewindeansatz umfasst/umfassen.

3. Medizinisches Vorrichtungssystem nach einem der Ansprüche 1-2, wobei der Katheterschaft ein Verstärkungselement aufweist.

4. Medizinisches Vorrichtungssystem nach einem der Ansprüche 1-3, wobei der entfernbare Liner ein Verstärkungselement aufweist.

5. Medizinisches Vorrichtungssystem nach einem der Ansprüche 1-4, wobei der Ersatzliner ein Lumen definiert und wobei das Stützelement einen im Lumen angeordneten Abgabemandrin aufweist.

6. Medizinisches Vorrichtungssystem nach einem der Ansprüche 1-4, wobei das Stützelement eine abnehmbare Hülle umfasst, die entlang einer Außenfläche des Ersatzliners angeordnet ist.

7. Medizinisches Vorrichtungssystem nach einem der Ansprüche 1-6, wobei der entfernbare Liner, der Ersatzliner oder beide zum Wechsel zwischen einer eingefahrenen Konfiguration und einer nicht-eingefahrenen Konfiguration ausgelegt ist/sind.

8. Medizinisches Vorrichtungssystem nach einem der Ansprüche 1-7, wobei der entfernbare Liner, der Ersatzliner oder beide ein temporäres distales Dichtungselement umfasst/umfassen.

9. Medizinisches Vorrichtungssystem nach einem der Ansprüche 1-8, wobei:
das erste Verbindungsstück mit einer proximalen Endregion des Katheterschafts gekoppelt ist;
das zweite Verbindungsstück mit dem entfernbaren Liner gekoppelt ist; und
das erste und das zweite Verbindungsstück zum lösbaren Verschachteln ineinander ausgelegt sind.

10. Medizinisches Vorrichtungssystem nach einem der Ansprüche 1-9, wobei der entfernbare Liner frei von einer Befestigung am Katheterschaft ist.

11. Medizinisches Vorrichtungssystem nach einem der Ansprüche 1-10, wobei der entfernbare Liner eine gleitfähige Beschichtung umfasst.

## Revendications

1. Système de dispositif médical, comprenant :
une tige de cathéter (16) ayant une extrémité proximale ;
un manchon amovible (18) qui s'étend dans la tige de cathéter (16),
où la tige de cathéter (16) inclut un premier embout (20) et où le manchon amovible inclut un second embout (22) conçu pour coopérer avec le premier embout ;
où le manchon amovible est conçu pour fournir une substance médicale à une région cible ;
où le manchon amovible est conçu pour être retiré de la tige de cathéter si le manchon amovible devient obturé ;
un manchon de remplacement (18') ;
où le manchon de remplacement est conçu pour être inséré dans la tige de cathéter après le retrait du manchon amovible ; et
un élément de support (32a) disposé le long du manchon de remplacement.

2. Système de dispositif médical selon la revendication 1, où le premier embout, le second embout, ou les deux, incluent un connecteur fileté.

3. Système de dispositif médical selon l'une quelconque des revendications 1-2, où la tige de cathéter inclut un élément de renforcement.

4. Système de dispositif médical selon l'une quelconque des revendications 1-3, où le manchon amovible inclut un élément de renforcement.

5. Système de dispositif médical selon l'une quelconque des revendications 1-4, où le manchon de remplacement définit une lumière et où l'élément de support inclut un stylet de fourniture disposé dans la lumière.

6. Système de dispositif médical selon l'une quelconque des revendications 1-4, où l'élément de support inclut une gaine de rupture disposée le long d'une surface externe du manchon de remplacement.

7. Système de dispositif médical selon l'une quelconque des revendications 1-6, où le manchon amovible, le manchon de remplacement, ou les deux, sont conçus pour se déplacer entre une configuration affaissée et une configuration non affaissée.

8. Système de dispositif médical selon l'une quelconque des revendications 1-7, où le manchon amovible, le manchon de remplacement, ou les deux, incluent un élément d'étanchéité distal temporaire.

9. Système de dispositif médical selon l'une quelconque des revendications 1-8, où :
le premier embout est couplé à une région d'extrémité proximale de la tige de cathéter ;
le second embout est couplé au manchon amovible ; et
où le premier embout et le second embout sont conçus pour être emboîtés de manière détachable l'un avec l'autre.

10. Système de dispositif médical selon l'une quelconque des revendications 1-9, où le manchon amovible est dépourvu de fixation avec la tige de cathéter.

11. Système de dispositif médical selon l'une quelconque des revendications 1-10, où le manchon amovible inclut un revêtement lubrifiant.
